# EUROPEAN PATENT SPECIFICATION

(11) **EP 4 478 974 B1**
(45) Date of publication and mention of the grant of the patent: **08.04.2026**
(21) Application number: 23703613.2
(22) Date of filing: 13.02.2023
(51) Int. Cl.: A61B 18/20

(54) **LIGHT TREATMENT DEVICE**
LICHTBEHANDLUNGSVORRICHTUNG
DISPOSITIF DE TRAITEMENT À LUMIÈRE

(30) Priority: 17.02.2022 EP 22157361
(43) Date of publication of application: 25.12.2024
(73) Proprietor: Koninklijke Philips N.V., 5656 AG Eindhoven (NL)
(72) Inventor: BOAMFA, Marius Iosif, 5656 AG Eindhoven (NL); VAN ABEELEN, Frank Anton, 5656 AG Eindhoven (NL); VERHAGEN, Rieko, 5656 AG Eindhoven (NL); JUTTE, Petrus Theodorus, 5656 AG Eindhoven (NL); PALERO, Jonathan Alambra, 5656AG Eindhoven (NL)
(74) Representative: Philips Intellectual Property & Standards
(86) International application number: PCT/EP2023/053449
(87) International publication number: WO 2023/156325

(56) References cited:
- WO-A1-2017/159342
- FR-A1- 2 871 290
- US-A- 5 498 933
- US-A1- 2007 247 080
- US-A1- 2009 046 223

## Description

### FIELD OF THE INVENTION

This disclosure relates to a treatment device for performing a light-based treatment operation on or to a subject.

### BACKGROUND OF THE INVENTION

Techniques for removal of unwanted hairs include shaving, electrolysis, plucking, laser and light therapies (known as photoepilation) and injection of therapeutic anti-androgens. Light-based technologies are also used in other types of dermatological treatments, including hair growth reduction and treating acne.

Light-based hair treatments inhibit the growth of hair by exposing the skin to bright flashes or pulses of light, known as intense pulsed light (IPL). Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. The IPL may be generated by a high intensity light source such as a gas discharge lamp (e.g., a Xenon flash lamp). The light penetrates the skin and is absorbed, among other places, in the root of the hair by the pigment melanin. This causes an increase in the temperature of the root of the hair to rise and subsequently the temperature of the surrounding tissue. The generated heat damages the hair follicles, and the growth of the hair is inhibited if the temperature rise is sufficient. This process is known as photothermolysis.

Light-based hair removal can be performed using commercially available 'home-use' devices (i.e. consumer devices that are suitable for use by non-specialists) such as the Philips Lumea device. Home-use devices typically use IPL technology at a relatively low fluence (e.g. up to 6.5J/cm²) compared to professional devices that use fluences in excess of 10J/cm². When the treatment is repeated in intervals of 2 to 4 weeks, a long-lasting hair reduction is achieved.

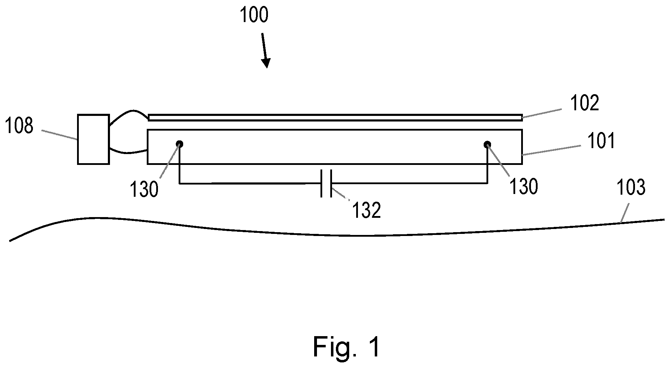

A discharge lamp suitable for light-based treatments is disclosed in US2007/247080 A1, for instance. Fig. 1 is a schematic depicting some of the components of an IPL treatment device 100. The device 100 comprises a gas discharge lamp 101 for generating an intense light pulse (also referred to herein as a flash) and a light reflector 102 positioned to guide the light pulse towards the skin 103 of the subject. The gas discharge lamp 101 comprises a gas in a housing, e.g. a glass tube. The gas is typically a noble gas, such as Xenon or Argon, or a mixture of such gases. The flash is generated by applying a voltage across the gas to cause an electric discharge. The voltage may be applied, for example, via internal electrodes 130 inside either end of the glass tube. The internal electrodes 130 are connected to one or more capacitors 132 (that are external to the glass tube) that store the electrical energy required for the voltage to be applied across the internal electrodes 130. In most cases, an ignition voltage (known as a trigger) is first required to (partially) ionise the gas inside the lamp before the main electrical pulse initiates the main discharge that generates the flash. The partial ionisation of the gas is referred to as plasma breakdown. In some cases, the ignition voltage can be applied between an electrode of the lamp (typically one of the two internal electrodes 130) and an additional external electrode that is outside the glass tube (known as external triggering). The additional external electrode (also referred to herein as the ignition electrode or "first external electrode") can be any electrically conductive component, e.g. a metallic component. In the example shown in Fig. 1, the reflector 102, which is a metallic component, is used as the additional external electrode such that the ignition voltage is applied between an electrode of the lamp 101 and the reflector 102. In another example, the additional external electrode could be a metallic component that is wound around the glass tube. The metallic component may be in the form of a wire or strip. In Fig. 1, the ignition voltage between the lamp 101 and the reflector 102 is generated by a plasma ignition unit 108.

Treatment devices 100 may include various sensors that are not shown in Fig. 1. For example, the standard devices currently on the market typically include a skin contact sensor and a skin colour or skin tone sensor. These sensors are included, in part, for safety purposes. The skin colour/tone sensor is used to regulate the pulse energy according to the colour/tone of the area of the subject's skin, e.g., so that less energy is used for darker skin tones. The skin contact sensor is used to ensure that the flash is enabled only when the device is correctly in contact with the treatment area. These and other sensors may be incorporated into the treatment device or provided as attachments for the device.

Imaging sensors (e.g. in a camera) are also increasingly being included in treatment devices 100 to obtain images of the area that is to be treated and/or that has been treated. Indeed, future standards and regulations appear to be evolving towards the inclusion of an imaging sensor in the IPL optical engine. The imaging sensor may be used to image the treatment area before the IPL flash is deployed to determine whether it is safe to perform the treatment on the imaged area. This could include detecting the skin tone of the area, the condition of the skin, and the presence of any tattoos, moles and/or other skin features that could impact on the suitability and/or safety of the light-based treatment. Other potential uses of an imaging sensor embedded in the treatment device could include treatment guidance via displacement measurement and evaluating the effects of a treatment.

### SUMMARY OF THE INVENTION

The invention and its most advantageous embodiments are defined in the appended set of claims. The high ignition voltage used to initiate the plasma breakdown in IPL treatment devices can put sensors and/or metallic components that are incorporated into the device near to the gas discharge lamp at risk. The size of the voltage that is required to ionise the gas in a gas discharge lamp is determined by the type of gas and the pressure of the gas. Typical ignition voltages for IPL devices are in region of 12 to 17 kV. At these voltages, nearby sensors/components are prone to capacitive coupling with the ignition electrode (i.e. the additional external electrode outside the glass tube) as well as being at risk of a direct voltage discharge from the ignition electrode to the sensor/component.

This problem has particularly been observed with the cameras that are increasingly being incorporated into treatment devices. An imaging sensor in an IPL device is preferably positioned close to the gas discharge lamp so that images of the area to be/being treated can be obtained. However, placing the imaging sensor close to the gas discharge lamp leads to capacitive coupling between the imaging sensor and the ignition electrode which can prevent the normal functioning of the imaging sensor. A direct discharge from the high voltage ignition electrode to the imaging sensor will also damage the imaging sensor and could lead to camera failure.

Therefore, it is desirable to provide a treatment device that reduces the risk of electrical damage within the device due to the high voltage used to initiate plasma breakdown in the gas discharge lamp. Such a treatment device will be more suitable for the incorporation of additional sensors and components (e.g., cameras) than the treatment devices that are currently available. Thus, it is an object of the present disclosure to provide a light-based treatment device in which an imaging sensor, as well as other types of sensors or electrical components, can be incorporated close to the gas discharge lamp without compromising the functionality of the device and/or the sensor(s)/component(s).

According to the disclosure herein, it is proposed to position an earthed electrode within the treatment device and close enough to (but not in contact with) the ignition electrode to provide a path to electrical ground. Thus, each time the ignition voltage is applied to enable a light pulse to be generated, a controlled, high voltage discharge takes place through the earthed electrode. It has been found that this controlled discharge does not prevent the light source from emitting the light pulse.

According to a first aspect, there is provided a treatment device for performing a light-based treatment operation on or to a subject. The treatment device comprises a light source configured to generate a light pulse, the light source comprising a gas; a plasma ignition unit configured to apply a first voltage between the light source and a first electrode to initiate plasma breakdown of the gas in the light source; a light exit window through which the light pulse is emitted from the treatment device; and a second electrode connected to electrical ground. The second electrode is spaced from the first electrode such that the first electrode discharges via the second electrode when the first voltage is applied.

According to a second aspect, there is provided a treatment device for performing a light-based treatment operation on or to a subject. The treatment device comprises a light source configured to generate a light pulse, the light source comprising a housing, a gas in the housing and two internal electrodes inside the housing. The treatment device also comprises a plasma ignition unit configured to apply a first voltage between the light source and a first external electrode that is external to the housing to initiate plasma breakdown of the gas in the light source; a capacitor configured to store electrical energy and to apply the stored electrical energy to the two internal electrodes to generate the light pulse when plasma breakdown of the gas has been initiated; and a second external electrode that is external to the housing and connected to electrical ground. The second external electrode is spaced from the first external electrode such that the first voltage at the first external electrode discharges via the second external electrode when the first voltage is applied.

Thus, the present invention provides a treatment device in which the risk of electrical damage to sensors and/or other electrical components that are within the device is reduced. This is achieved without compromising the functionality of the treatment device and/or the sensor(s) therein. The invention can enable sensors such as imaging sensors to be positioned close to the light source so that the imaging sensor can obtain images of the area being treated with the light pulse. Alternatively or in addition, the invention can provide greater freedom in designing treatment devices, since sensors and/or electrical components can be positioned closer to the light source than in conventional treatment devices. The earthed electrode provides for a controlled (and predictable) discharge during every light pulse and prevents an uncontrolled discharge that could damage nearby sensors/components. The present disclosure also reduces the length of time that capacitive coupling between the ignition electrode and sensor(s) in the device takes place.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic showing some of the components of a treatment device;
Fig. 2 is an illustration of an exemplary treatment device;
Fig. 3 is a schematic demonstrating the problem that can arise in treatment devices;
Fig. 4 is a schematic of parts of a treatment device according to various embodiments;
Fig. 5 is a schematic of parts of a treatment device according to various embodiments;
Fig. 6 is a schematic of a case arrangement for an imaging sensor according to various embodiments;
Fig. 7 is a schematic of the case arrangement of Fig. 6 in part of a treatment device according to various embodiments; and
Fig. 8 is a schematic of parts of a treatment device according to various embodiments.

### DETAILED DESCRIPTION OF THE EMBODIMENTS

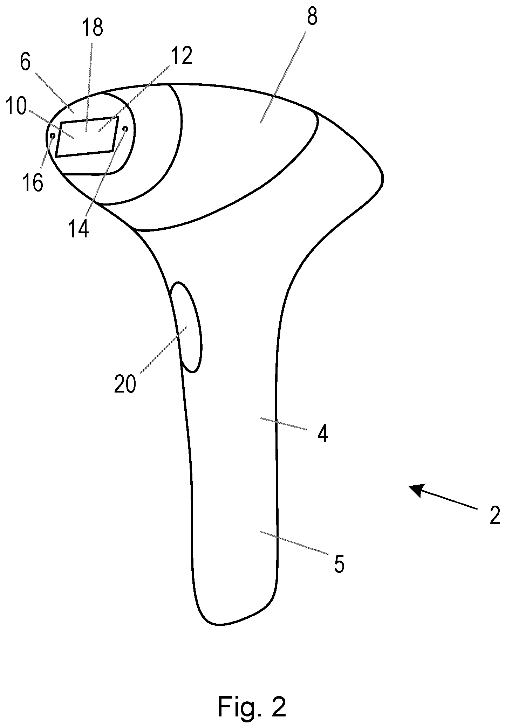

Fig. 2 is an illustration of an exemplary treatment device 2 that can be used to apply a light pulse to an area of skin. It will be appreciated that the treatment device 2 in Fig. 2 is merely presented as an example of a hand-held treatment device 2 that the invention can be used with, and the treatment device 2 is not limited to the form shown in Fig. 2 or to being a hand-held treatment device. The treatment device 2 is for use on a body of a subject (e.g. a person or an animal), and is to be held in one or both hands of a user during use. The treatment device 2 is to perform some treatment operation to hairs on the body of the subject using one or more light pulses when the treatment device 2 is in contact with a body part of the subject. The treatment operation can be removal of unwanted hairs by laser and/or light therapies (known as a photoepilation treatment or Intense Pulsed Light treatment).

As described herein, the treatment device 2 is operated or used by a 'user', and the treatment device 2 is used on a body of a 'subject'. In some cases, the user and the subject is the same person, i.e. the treatment device 2 is held in a hand and used by a user on themselves (e.g. used on the skin on their leg). In other cases, the user and the subject are different people, e.g. the treatment device 2 is held in a hand and used by a user on someone else.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed into contact with the subject in order for the personal care operation to be performed on the body or skin of the subject at the position that the head portion 6 is in contact with the body or skin.

The treatment device 2 is for performing a treatment operation using light pulses. Thus, in Fig. 2 the head portion 6 comprises an aperture 10, also referred to as a light exit window, that is arranged in or on the housing 4 so that the aperture 10 can be placed adjacent to or on (i.e. in contact with) the skin of the subject. The treatment device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10. The aperture/light exit window 10 may be in the form of an opening at the head end 8 of the housing 4, or it may be in the form of a window (including a waveguide) that is transparent or semi-transparent to the light pulses (i.e. the light pulses can pass through the window).

In the exemplary embodiment shown in Fig. 2, the aperture 10 has a generally rectangular shape, which results in a generally rectangular-shaped skin treatment region on the skin. It will be appreciated that the aperture 10 can have any other desired shape. For example the aperture 10 can be square, elliptical, circular, or any other polygonal shape.

The one or more light sources 12 can generate light pulses of any suitable or desired wavelength (or range of wavelengths) and/or intensities. For example, the light source 12 can generate visible light, infra-red (IR) light and/or ultraviolet (UV) light. Each light source 12 can comprise any type of light source for which a high voltage is to be applied in order to enable a light pulse to be generated, such as a gas discharge lamp.

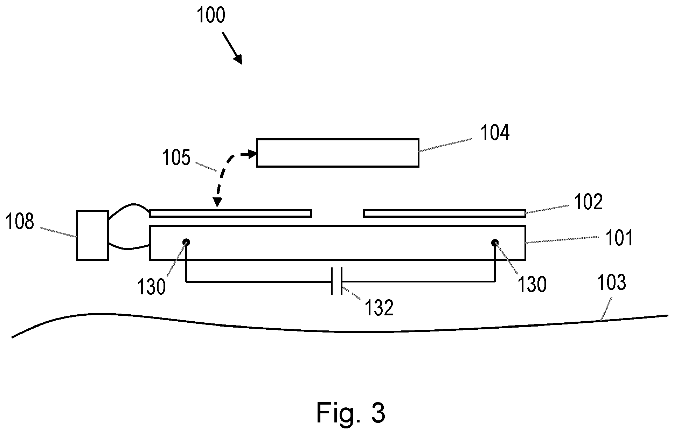

According to the embodiments herein, at least one of the one or more light sources is a gas discharge lamp (e.g. a Xenon flash lamp). The gas discharge lamp may comprise a gas in a housing (e.g. a glass tube), where the gas is typically a noble gas, such as Xenon or Argon, or a mixture of such gases. In these embodiments, the treatment device 2 also includes a plasma ignition unit for providing a high voltage to initiate plasma breakdown of the gas in the light source. This process is further described with reference to Fig. 3.

In some embodiments, the light source(s) 12 generate UV, visible and/or IR light, and a filter may be used to absorb some wavelengths. For example, the filter may remove UV light and visible light at the blue end of the spectrum. In some embodiments, light with wavelengths of less than 560 nm may be filtered out. The resulting light pulse(s) may be emitted from the device with spectral content in the 560-1800 nanometre (nm) range. These wavelengths (in particular wavelengths in the range 560 to 1200 nm) heat melanin in the hair and hair root by absorption, which puts the hair follicles in a resting phase, preventing hair regrowth.

The one or more light sources 12 are configured to provide pulses of light. That is, the light source(s) 12 are configured to generate light at a high intensity for a short duration (e.g. less than 1 second). In some examples, the light pulse may have a duration between 4 and 8 milliseconds (ms). The Full Width Half Maximum (FWHM) of the light pulse may be about 1.8 ms. The intensity of the light pulse should be high enough to effect the treatment operation on the skin or body part adjacent the aperture 10. For example, home-use devices can provide light pulses at fluences of up to 6.5 J/cm², while professional devices can provide light pulses at fluences in excess of 10 J/cm².

The illustrated treatment device 2 also includes two skin contact sensors 14, 16 positioned on or in the head portion 6 that are used to determine whether the head portion 6 is in contact with the skin before a light pulse is generated to avoid the light pulse being directed into the eyes of the user or subject.

The illustrated treatment device 2 includes an imaging sensor 18 that is located in the head portion 6 and is positioned close to one or more of the light source(s) 12. In some embodiments, the imaging sensor 18 can be configured to obtain images of the area of skin that: is to be treated, is being treated or has been treated. For example, the imaging sensor 18 may be used to determine the condition of the skin to be treated and/or whether the treatment area includes any tattoos, moles and/or other skin features that could impact on the suitability and/or safety of the light-based treatment.

Alternatively, or in addition, the imaging sensor 18 may be configured to determine a skin tone of the skin that is to be treated. The skin tone may be determined to make sure that the light pulse has an intensity that is appropriate for the type of skin being treated, or even to prevent a light pulse being generated if the skin type is unsuitable for light pulses (e.g. darker skin which has a much higher melanin content). The imaging sensor 18 may determine the skin tone by measuring an intensity or level of light at a particular wavelength or multiple wavelengths reflected from the skin. The measured intensity or level of reflected light at a particular wavelength(s) can be indicative of the skin tone. The measured intensity or level of reflected light can be based on the concentration of melanin in the skin, and thus the measured intensity or level can indicate the melanin concentration. The melanin concentration can be derived, for example, from measurements of light reflection at 660nm (red) and 880nm (infrared) wavelengths.

In some embodiments, the imaging sensor 18 may be configured to determine whether the head portion 6 of the device is in contact with the skin of a subject. **In** these embodiments, the imaging sensor 18 may be used instead of, or as well as, the skin contact sensors 14, 16 described above.

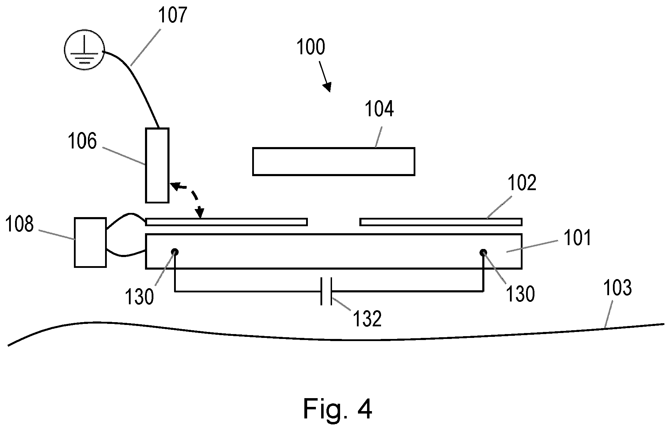

The imaging sensor 18 may be, for example, the imaging sensor 104 described with reference to any of Figs. 4-6.

The illustrated treatment device 2 also includes a user control 20 that can be operated by the user to activate the treatment device 2 so that the head portion 6 performs the required treatment operation on the body of the subject (e.g. the generation of one or more light pulses by the one or more light source(s) 12). The user control 20 may be in the form of a switch, a button, a touch pad, etc.

As noted above, there are challenges associated with incorporating an imaging sensor and/or other sensors in IPL treatment devices such as those depicted in Figs. 1 and 2. These challenges are described with reference to Fig. 3.

The part of the treatment device 100 that was depicted in Fig. 1 is shown in Fig. 3 with an imaging sensor 104 positioned close to the lamp 101 and the reflector 102. There is an opening in the reflector 102 so that the imaging sensor 104 can obtain images of the skin 103 of a subject via a light exit window (not shown) and the (e.g. glass) housing of the lamp 101. In this example, the lamp 101 is a gas discharge lamp, such as a Xenon flash lamp. Two internal electrodes 130 are present inside the housing of the lamp 101 that can be connected to one or more capacitors 132. The capacitor(s) 132 are external to the housing (glass tube) of the lamp 101 and store the electrical energy required to apply the voltage across the internal electrodes 130 to generate the flash. In practice at least part of each internal electrode 130 is inside the glass tube, and they can extend to outside the glass tube where they connect to the capacitor(s) 132.

As described with reference to Fig. 1, the device 100 comprises a plasma ignition unit 108 that is configured to, at the beginning of every flash, apply an ignition voltage between the reflector 102 and an electrode of the lamp 101, for example one of the two internal electrodes 130 (that form an open circuit). The ignition voltage must be sufficiently high to form a plasma, i.e., to ionise the gas atoms and/or molecules in the gas discharge lamp 101. For typical IPL devices, the ignition voltage is in the range 5 to 30 kV. Preferably, the ignition voltage is in the range 12 to 17 kV. In some examples, the ignition voltage is 15 kV.

When the reflector 102 is placed under high voltage (e.g. when the reflector 102 is acting as an external electrode for the ignition voltage), capacitive coupling occurs between the reflector 102 and any electrically conductive components in its vicinity. Therefore, the configuration shown in Fig. 3 leads to capacitive coupling 105 between the reflector 102 and the imaging sensor 104. The imaging sensor 104 is also at risk of receiving a direct discharge from the reflector 102. Such capacitive coupling and/or voltage discharge (depicted in Fig. 3 by the dashed arrow 105) can prevent the normal functioning of the imaging sensor and can lead to short- or long-term damage to the imaging sensor comprised therein.

To address these and other problems, there is provided a treatment device for performing a light-based treatment operation on or to a subject. The treatment device comprises: a light source comprising a housing, a gas in the housing and two internal electrodes inside the housing; a plasma ignition unit configured to apply a first voltage between the light source and a first external electrode that is external to the housing to initiate plasma breakdown of the gas in the light source; a capacitor configured to store electrical energy and to apply the stored electrical energy to the two internal electrodes to generate the light pulse when plasma breakdown of the gas has been initiated; and a second external electrode that is external to the housing and connected to electrical ground. The second external electrode is spaced from (i.e. not in contact with) the first external electrode such that the first voltage at the first external electrode discharges via the second external electrode when the first voltage is applied.

The treatment device may be an Intense Pulsed Light, IPL, device for performing IPL photo-epilation. The light source may be a gas discharge lamp such as a flash lamp. Examples of gases used in flash lamps include a Xenon, Argon and Krypton. In some embodiments, the flash lamp may comprise a molecular gas and/or a mixture of gases.

The light source may comprise a housing (e.g. a glass tube) in which the gas is contained. The light source further comprises at least two internal electrodes (distinct from the first and second external electrodes already mentioned) that are spaced from each other such that there is gas between them. The two internal electrodes are at least partly arranged inside the housing of the lamp and are connected to one or more capacitors that are external to the housing. The capacitor(s) store the electrical energy required to apply the voltage across the internal electrodes to generate the flash. A switching device may be present to control the discharge of the one or more capacitors over the two internal electrodes.

The plasma ignition unit is configured to apply a first voltage (also referred to herein as an ignition voltage) between the light source and the first external electrode (also referred to herein as an ignition electrode) to initiate plasma breakdown of the gas in the light source. The first voltage may be between one of the internal electrodes 130 in the lamp 101 and the first external electrode. Preferably the internal electrode 130 used here is the negative electrode. In some embodiments, the first voltage is between 5 and 30 kV. Preferably, the first voltage is between 12 and 17 kV.

The first external electrode can be any suitable electrically conductive component. For example, in some embodiments the first electrode is part of a reflector that is also used for guiding the light pulse to the light exit window. In other embodiments, the first external electrode is part of a metal component (e.g. a metal wire) that is wound around the light source.

The first external electrode is comprised in the treatment device. In some embodiments, the first external electrode is positioned outside the light source for external triggering of the light source. In these embodiments, the first external electrode may be in contact with the light source (e.g. in contact with the housing (e.g. glass tube)), or spaced from, but proximal to (i.e. in the close vicinity of) the light source. In some embodiments, 'proximal to' could mean spaced from the light source by between 0.1 and 2 mm. The first external electrode is fully outside/external to the housing/glass tube of the lamp 101.

Although not shown in Fig. 3, the treatment device comprises a light exit window through which the light pulse is emitted from the treatment device. The light exit window may be an aperture, e.g. a hole/opening in the housing of the treatment device. Alternatively, the light exit window may be a translucent or transparent material (e.g. glass or plastic) through which light can travel.

In some embodiments, the treatment device further comprises a sensor. For example, the sensor could be an imaging sensor for obtaining one or more images of an area or region near to the treatment device, for example an area of skin that is to be treated using the light pulse or an area of skin that has been treated using the light pulse. The imaging sensor obtains one or more images using light passing into the treatment device. Alternative types of sensors include a skin contact sensor or a skin tone sensor. The sensor could be for sensing one or more of: skin contact, skin colour, hair colour, moles, tattoos, and skin condition. In the case of an imaging sensor, the image(s) obtained by the imaging sensor can be analysed to determine any one or more of skin contact, skin colour, hair colour, moles, tattoos, and skin condition.

In embodiments where a sensor is present in the treatment device, at least a part of the second external electrode is closer to the first external electrode than any part of the sensor. In other words, at least a part of the second external electrode is spaced from the first external electrode by a smaller distance than the smallest distance between the sensor and the first external electrode. This ensures that the first voltage discharges to ground via the second external electrode and not the sensor.

In some embodiments, the smallest distance between the second external electrode and the first external electrode is equal to or smaller than an air breakdown distance for the first voltage at atmospheric pressure. This positioning reduces the risk of electrical damage to the sensor. Each time the first (ignition) voltage is applied, a high voltage discharge takes place to the earthed second external electrode. This reduces the length of time of capacitive coupling into the sensor. Furthermore, the high voltage discharge to the earthed second external electrode prevents a high voltage discharge to the sensor.

The inclusion of the second external electrode provides for a controlled voltage discharge each time a flash in generated. The ignition voltage (e.g. approximately 15 kV) is required to initiate plasma breakdown in the flash lamp. As such, it may be expected that a controlled discharge of this voltage would compromise the normal functioning of the flash lamp. In other words, it may be expected that if the ignition voltage discharges, then the ignition voltage will not be present to (partially) ionise the gas in the light source (i.e. to initiate plasma breakdown), and therefore the flash will not be generated. However, experimental observation shows that the controlled discharge does not prevent the emission of the intense light pulse, provided the earthed second external electrode is not in conductive contact with the ignition first external electrode (i.e. provided there is not a conductive path between the ignition first external electrode and the earthed second external electrode). The experimental observations indicate that if the ignition first external electrode is spaced from the earthed second external electrode, the time needed to initiate plasma breakdown is less than the time needed for the controlled discharge of the ignition voltage. Therefore, the controlled discharge does not prevent plasma breakdown. Conversely, if the ignition first external electrode is in conductive contact with the earthed second external electrode, then the ignition voltage discharges directly to ground, and no plasma breakdown can occur, thereby preventing the generation of the IPL flash.

As disclosed herein, the inclusion of an earthed second external electrode that is spaced from the first external electrode provides a path for current to flow to ground in a controlled manner. This controlled discharge prevents the imaging sensor or any other components in the device from becoming a high voltage discharge path and thus protects such components from electrical damage. The earthed second external electrode also reduces the length of time of any capacitive coupling between: (i) the ignition first external electrode in the lamp, and (ii) the imaging sensor or other sensor(s)/component(s). As such, sensors (such as an imaging sensor) and/or other components can be included in the treatment device with a reduced risk of electrical damage to the sensors/components, and without compromising the functionality of the treatment device.

Specific embodiments of the invention are depicted schematically in Figs. 4, 5, 6, 7 and 8.

Fig. 4 shows a treatment device 100 with an imaging sensor 104 positioned close to the lamp 101 and the reflector 102. Two internal electrodes 130 are present inside the housing of the lamp 101 (e.g. inside the glass tube) and are connected to one or more capacitors 132. The capacitor(s) 132 store the electrical energy required to apply the voltage across the internal electrodes 130 to generate the treatment light pulse.

The imaging sensor is configured to obtain one or more images using light passing into the treatment device. The obtained images could be, for example, images of the skin 103 before it is to be treated and/or after it has been treated. It will be understood that the invention is equally applicable to other sensors or components in place of, or in addition to, the imaging sensor 104.

The reflector 102 in Fig. 4 has a dual function. Firstly, it reflects the treatment light pulse towards the skin 103 of a subject. Secondly, it acts as a high voltage first external electrode for the ignition voltage that is used to initiate plasma breakdown in the lamp 101. The treatment device 100 also comprises a second external electrode 106 that is connected to electrical earth 107. The second external electrode 106 is spaced from the reflector 102 (first external electrode) such that, each time the plasma ignition unit 108 applies the first voltage (i.e. the ignition voltage) between the reflector 102 and the lamp 101, the high voltage in the reflector 102 discharges via the second external electrode 106. It has been experimentally observed that this high voltage discharge to an earthed second external electrode 106 does not prevent the proper functioning of the lamp 101 and, thus, does not prevent the IPL emission.

The smallest distance between the second external electrode 106 and the reflector 102 (first external electrode) is equal to or smaller than an air breakdown distance for the first voltage at atmospheric pressure. This ensures that every time the ignition voltage is applied, a high voltage discharge to the second external electrode 106 takes place.

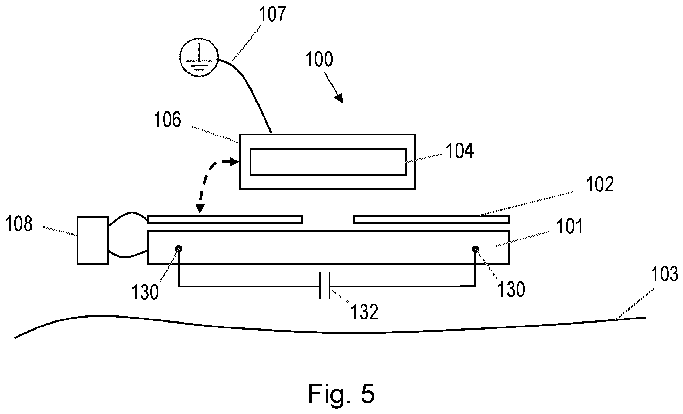

Fig. 5 shows an alternative embodiment in which the second external electrode 106 is a case or housing in which the imaging sensor 104 is disposed, e.g., a metallic case around a camera. In other embodiments, the second external electrode 106 could be a case in which any other type of sensor is disposed. The case 106 is connected to electrical earth 107, electrically isolated from the imaging sensor 104 contained therein, and is spaced from the reflector 102 (first external electrode) such that, each time the first voltage (i.e. the ignition voltage) is applied between the reflector 102 and the lamp 101, the reflector 102 discharges via the second external electrode 106. The current flows to ground in a controlled manner and therefore does not pass through the imaging sensor 104 (or other sensor(s)).

A drawback of integrating an imaging sensor 104 in the path of the treatment light in a treatment device 100 is the possibility of electromagnetic interference (EMI) from the ignition of the lamp 101. For example, a high voltage (e.g. 15 kV) may be used on the reflector 102 (first external electrode) behind the flash lamp 101 to initiate the plasma channel inside the lamp 101. EMI is a disturbance generated by an external source that affects an electrical circuit by electromagnetic induction, electrostatic coupling, or conduction. The disturbance may degrade the performance of the imaging unit 104 or even stop it from functioning.

Although the second external electrode 106 can be a metallic case or housing in which the imaging sensor 104 is disposed, and in principle the imaging sensor 104 can be well-shielded from EMI in this solution, the case or housing requires an opening to enable the imaging sensor 104 to obtain images, and substantial EMI can occur through the unshielded optical path through the opening to the imaging unit 104.

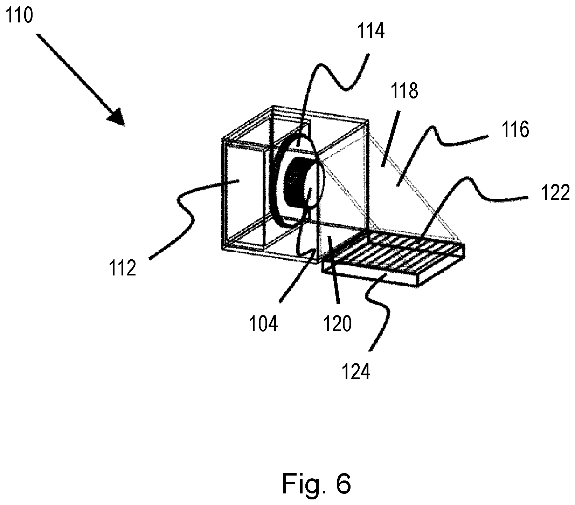

An exemplary embodiment of a case arrangement 110 for an imaging sensor 104 that improves the EMI shielding is shown in Fig. 6. The case arrangement 110 has the imaging sensor 104 housed in an electrically conductive housing 112 that acts as the second external electrode 106. In this illustrated embodiment, the imaging sensor 104 has an LED light illumination ring 114 that is used to generate light for illuminating the skin 103 to be imaged. The illumination ring 114 may consist of a finite number of LEDs, e.g. 3 or 4. In other embodiments of the case arrangement 112, the illumination ring 114 can be omitted, or located in a different part of the treatment device 100.

The electrically conductive housing 112 is in the form of an open-ended housing, e.g. a box with one open end, through which the imaging unit 104 obtains images. A metal-coated triangular prism 116, for example made of glass or optical plastic, reflects the light from the illumination ring 114 (which is referred to as the 'illumination beam') in the downward direction to the exit side of the prism 116. The metal coating is applied to the hypotenuse surface 118 of the prism 116, where it acts as both an EMI shield, and a mirror for the illumination beam and light reflected back from the skin 103. The metal coating is also applied to the triangular side surfaces 120, which act as an EMI shield. The exit surface of the prism 116 is covered with a conductive (e.g. metal) nanowire grid 122 that acts as an optical polarizer. The direction of the nanowire grid 122 is preferably aligned parallel to the long axis of the treatment device 100 aperture/light exit window 10 to ensure the highest angular performance in that direction. Optionally, the light from the illumination ring 114 is pre-polarized by an absorbing or reflecting polarizer film to prevent excessive internal reflectance from the wire-grid polarizer 122 back into the imaging sensor 104. In front of the nanowire grid polarizer 122 can be a plate of achromatic λ/4 (i.e. quarter wavelength) retarder 124 that converts the linearly polarized light into circularly polarized light on emission, while specularly reflected light will have undergone an 180° phase switch leading to cross-polarized light on return, preventing specular reflections entering the imaging sensor 104. The light that passes the polarizer filter is reflected towards the imaging sensor 104 by the hypotenuse surface 118 of the prism 116.

Optionally, the electrically conductive housing 112 is constructed so that it has a high external reflectance. This promotes reflection of the treatment light pulse when it encounters the housing 112 around the imaging sensor 104, thereby resulting in minimal loss in optical power at the aperture/light exit window 10.

The electrically conductive housing 112 may be a glass (or plastic) hollow cylinder with a metal (e.g. silver/aluminum/copper) lining. In these embodiments, the glass (plastic) wall can provide mechanical support and double as an electric insulator between the Faraday cage formed by the electrically conductive housing 112 and the reflector 102 behind the flash lamp 101 to which the 15 kV ignition voltage is applied.

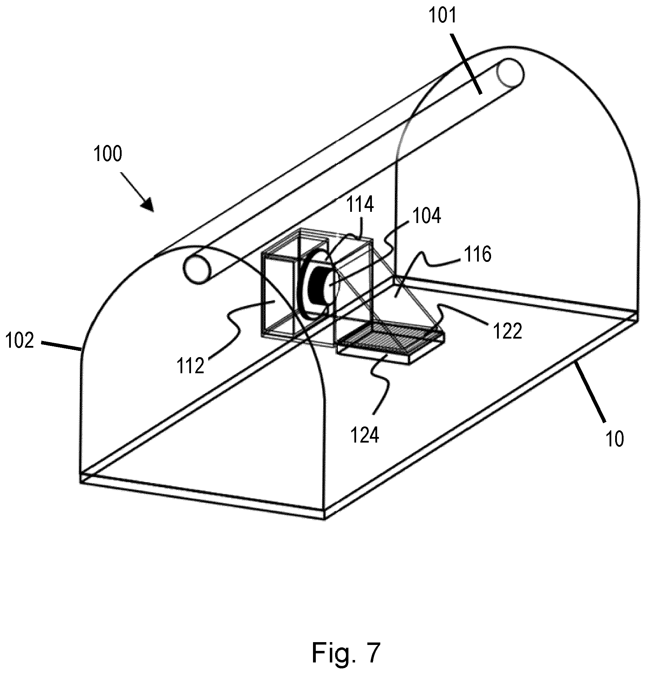

Fig. 7 is a schematic of the case arrangement 110 of Fig. 6 in part of a treatment device 100 according to various embodiments. The case arrangement 110 and imaging sensor 104 are shown inside an optical cavity in the treatment device 100 that is formed by the shape of the reflector 102, and bounded by the aperture/light exit window 10. The lamp 101 is also shown in Fig. 7. Thus the case arrangement 110 is arranged so that the plane of the nanowire grid polarizer 122 is parallel to the plane of the aperture/light exit window 10.

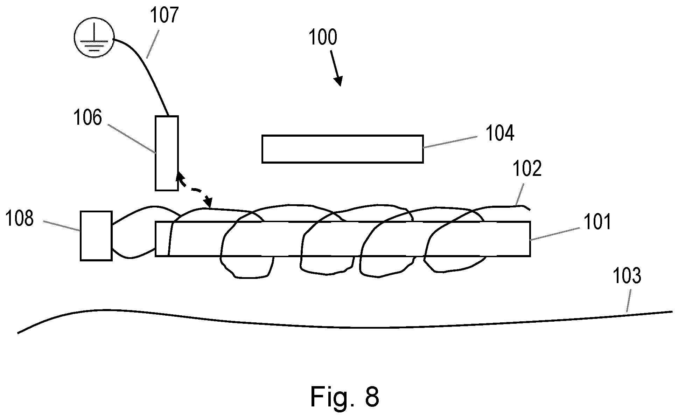

Fig. 8 shows an alternative embodiment of the first external electrode 102 in which the first external electrode is a metal wire 102 wound around the lamp 101. In this way, the metal wire 102 (or similar type of metallic component, such as a strip) can act as a first external electrode for the ignition voltage. Each time the plasma ignition unit 108 applies the ignition voltage between the lamp 101 and the metal wire 102, a controlled discharge takes place to the earthed second external electrode 106, thereby protecting the imaging sensor 104.

Therefore, there is provided a treatment device for performing a light-based treatment operation on or to a subject, where the treatment device is configured so that the risk of electrical damage to sensors and/or components in the vicinity of the light source comprised therein is reduced compared to conventional treatment devices.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A treatment device (2, 100) for performing a light-based treatment operation on or to a subject, the treatment device (2, 100) comprising:
a light source (12, 101) configured to generate a light pulse, the light source (12, 101) comprising a housing, a gas in the housing and two internal electrodes (130) inside the housing;
a plasma ignition unit (108) configured to apply a first voltage between the light source (12, 101) and a first external electrode (102) that is external to the housing to initiate plasma breakdown of the gas in the light source (12, 101);
a capacitor (132) configured to store electrical energy and to apply the stored electrical energy to the two internal electrodes (130) to generate the light pulse when plasma breakdown of the gas has been initiated; and
a second external electrode (106) that is external to the housing and connected to electrical ground;
wherein the second external electrode (106) is spaced from the first external electrode (102) such that the first voltage at the first external electrode (102) discharges via the second external electrode (106) when the first voltage is applied.

2. A treatment device (2, 100) as defined in claim 1, wherein the treatment device (2, 100) further comprises a sensor (104).

3. A treatment device (2, 100) as defined in claim 2, wherein the sensor (104) is an imaging sensor for obtaining one or more images using light passing into the treatment device (2, 100).

4. A treatment device (2, 100) as defined in claim 2 or 3, wherein the sensor (104) is for sensing one or more of: skin contact, skin colour, hair colour, moles, tattoos, and skin condition.

5. A treatment device (2, 100) as defined in any of claims 2-4, wherein the second external electrode (106) is a case (110) in which the sensor (104) is disposed.

6. A treatment device (2, 100) as defined in claim 5, wherein the case (110) comprises an electrically conductive housing (112) for the sensor (104) that provides shielding from electromagnetic interference, EMI, due to the first voltage on the first external electrode (102).

7. A treatment device (2, 100) as defined in claim 6, wherein the sensor (104) is an imaging sensor for obtaining one or more images using light passing into the treatment device (2, 100), wherein the electrically conductive housing (112) has an open end through which said light passes, and wherein the case (110) further comprises:
a triangular prism (116) with two triangular-shaped side surfaces (120), a first surface, a second surface and a hypotenuse surface (118), wherein the first surface is arranged adjacent to the open end of the electrically conductive housing (112) such that light entering the triangular prism (116) at the second surface reflects from the hypotenuse surface (118) towards the first surface, wherein the second surface is covered with a conductive nanowire grid (122) that acts as an optical polarizer to incident light, and wherein the hypotenuse surface (118) and the triangular-shaped side surfaces (120) are metal-coated to provide shielding of the imaging sensor from EMI due to the first voltage on the first external electrode (102).

8. A treatment device (2, 100) as defined in any of claims 2-7, wherein at least a part of the second external electrode (106) is closer to the first external electrode (102) than any part of the sensor.

9. A treatment device (2, 100) as defined in any of claims 2-8, wherein the sensor is arranged within the treatment device (2, 100) to sense one or more parameters of the subject via a light exit window (10) through which the light pulse is emitted from the treatment device (2, 100).

10. A treatment device (2, 100) as defined in any of claims 1-9, wherein the first external electrode (102) is a metallic component.

11. A treatment device (2, 100) as defined in any of claims 1-10, wherein the first external electrode (102) is a reflector for guiding the light pulse to the light exit window (10).

12. A treatment device (2, 100) as defined in any of claims 1-11, wherein the smallest distance between the second external electrode (106) and the first external electrode (102) is equal to or smaller than an air breakdown distance for the first voltage at atmospheric pressure.

13. A treatment device (2, 100) as defined in any of claims 1-12, wherein the first voltage is between 12 and 17 kV.

14. A treatment device (2, 100) as defined in any of claims 1-13, wherein the light source (12, 101) is a gas discharge lamp.

15. A treatment device (2, 100) as defined in claim 14, wherein the gas discharge lamp is a flash lamp.

16. A treatment device (2, 100) as defined in any of claims 1-15, wherein the treatment operation is Intense Pulsed Light, IPL, photo-epilation.

## Patentansprüche

1. Behandlungsvorrichtung (2, 100) zum Durchführen einer lichtbasierten Behandlung an oder bei einer Person, wobei die Behandlungsvorrichtung (2, 100) Folgendes umfasst:
eine Lichtquelle (12, 101), die so konfiguriert ist, dass sie einen Lichtimpuls erzeugt, wobei die Lichtquelle (12, 101) ein Gehäuse, ein Gas im Gehäuse und zwei interne Elektroden (130) im Inneren des Gehäuses umfasst;
eine Plasmazündeinheit (108), die so konfiguriert ist, dass sie eine erste Spannung zwischen der Lichtquelle (12, 101) und einer ersten externen Elektrode (102) anlegt, die sich außerhalb des Gehäuses befindet, um einen Plasmadurchschlag des Gases in der Lichtquelle (12, 101) einzuleiten;
einen Kondensator (132), der so konfiguriert ist, dass er elektrische Energie speichert und die gespeicherte elektrische Energie an die zwei internen Elektroden (130) anlegt, um den Lichtimpuls zu erzeugen, wenn der Plasmadurchschlag des Gases eingeleitet wurde; und
eine zweite externe Elektrode (106), die sich außerhalb des Gehäuses befindet und mit der elektrischen Masse verbunden ist;
wobei die zweite externe Elektrode (106) von der ersten externen Elektrode (102) so beabstandet ist, dass die erste Spannung an der ersten externen Elektrode (102) über die zweite externe Elektrode (106) entladen wird, wenn die erste Spannung angelegt wird.

2. Behandlungsvorrichtung (2, 100) nach Anspruch 1, wobei die Behandlungsvorrichtung (2, 100) weiter einen Sensor (104) umfasst.

3. Behandlungsvorrichtung (2, 100) nach Anspruch 2, wobei der Sensor (104) ein Bildsensor ist, der mit Hilfe von Licht, das in die Behandlungsvorrichtung (2, 100) eintritt, ein oder mehrere Bilder erhält.

4. Behandlungsvorrichtung (2, 100) nach Anspruch 2 oder 3, wobei der Sensor (104) zum Erfassen eines oder mehrerer der folgenden Merkmale dient: Hautkontakt, Hautfarbe, Haarfarbe, Muttermale, Tätowierungen und Hautzustand.

5. Behandlungsvorrichtung (2, 100) nach einem der Ansprüche 2-4, wobei die zweite externe Elektrode (106) ein Gehäuse (110) ist, in dem der Sensor (104) angeordnet ist.

6. Behandlungsvorrichtung (2, 100) nach Anspruch 5, wobei das Gehäuse (110) ein elektrisch leitfähiges Gehäuse (112) für den Sensor (104) umfasst, das eine Abschirmung gegen elektromagnetische Störungen, EMI, aufgrund der ersten Spannung an der ersten externen Elektrode (102) bereitstellt.

7. Behandlungsvorrichtung (2, 100) nach Anspruch 6, wobei der Sensor (104) ein Bildsensor zum Erhalten eines oder mehrerer Bilder mittels in die Behandlungsvorrichtung (2, 100) einfallendem Licht ist, wobei das elektrisch leitfähige Gehäuse (112) ein offenes Ende aufweist, durch das das Licht hindurchtritt, und wobei das Gehäuse (110) weiter Folgendes umfasst:
ein dreieckiges Prisma (116) mit zwei dreieckigen Seitenflächen (120), einer ersten Fläche, einer zweiten Fläche und einer Hypotenusenfläche (118), wobei die erste Fläche an das offene Ende des elektrisch leitfähigen Gehäuses (112) angrenzt, sodass Licht, das an der zweiten Fläche in das dreieckige Prisma (116) eintritt, von der Hypotenusenfläche (118) zur ersten Fläche reflektiert wird, wobei die zweite Fläche mit einem leitfähigen Nanodrahtgitter (122) bedeckt ist, das als optischer Polarisator für einfallendes Licht dient, und wobei die Hypotenusenfläche (118) und die dreieckigen Seitenflächen (120) metallbeschichtet sind, um den Bildsensor vor EMI durch die erste Spannung an der ersten externen Elektrode (102) abzuschirmen.

8. Behandlungsvorrichtung (2, 100) nach einem der Ansprüche 2-7, wobei mindestens ein Teil der zweiten externen Elektrode (106) näher an der ersten externen Elektrode (102) liegt als irgendein Teil des Sensors.

9. Behandlungsvorrichtung (2, 100) nach einem der Ansprüche 2-8, wobei der Sensor innerhalb der Behandlungsvorrichtung (2, 100) angeordnet ist, um einen oder mehrere Parameter der Person über ein Lichtaustrittsfenster (10) zu erfassen, durch das der Lichtimpuls von der Behandlungsvorrichtung (2, 100) ausgesendet wird.

10. Behandlungsvorrichtung (2, 100) nach einem der Ansprüche 1-9, wobei die erste externe Elektrode (102) ein metallisches Bauteil ist.

11. Behandlungsvorrichtung (2, 100) nach einem der Ansprüche 1-10, wobei die erste externe Elektrode (102) ein Reflektor ist, der den Lichtimpuls zum Lichtaustrittsfenster (10) leitet.

12. Behandlungsvorrichtung (2, 100) nach einem der Ansprüche 1-11, wobei der kleinste Abstand zwischen der zweiten externen Elektrode (106) und der ersten externen Elektrode (102) gleich oder kleiner als eine Luftdurchschlagsdistanz für die erste Spannung bei Atmosphärendruck ist.

13. Behandlungsvorrichtung (2, 100) nach einem der Ansprüche 1-12, wobei die erste Spannung zwischen 12 und 17 kV liegt.

14. Behandlungsvorrichtung (2, 100) nach einem der Ansprüche 1-13, wobei die Lichtquelle (12, 101) eine Gasentladungslampe ist.

15. Behandlungsvorrichtung (2, 100) nach Anspruch 14, wobei es sich bei der Gasentladungslampe um eine Blitzlampe handelt.

16. Behandlungsvorrichtung (2, 100) nach einem der Ansprüche 1-15, wobei es sich bei der Behandlung um eine Intense-Pulsed-Light-, IPL-, Photoepilation handelt.

## Revendications

1. Dispositif de traitement (2, 100) pour réaliser une opération de traitement par la lumière sur ou à un sujet, le dispositif de traitement (2, 100) comprenant :
une source de lumière (12, 101) configurée pour générer une impulsion de lumière, la source de lumière (12, 101) comprenant un logement, un gaz dans le logement et deux électrodes internes (130) à l'intérieur du logement ;
une unité d'allumage de plasma (108) configurée pour appliquer une première tension entre la source de lumière (12, 101) et une première électrode externe (102) qui est externe au logement pour amorcer la rupture du plasma du gaz dans la source de lumière (12, 101) ;
un condensateur (132) configuré pour stocker de l'énergie électrique et pour appliquer l'énergie électrique stockée aux deux électrodes internes (130) pour générer l'impulsion de lumière lorsque la rupture du plasma du gaz a été amorcée ; et
une seconde électrode externe (106) qui est externe au logement et connectée à la terre électrique ;
dans lequel la seconde électrode externe (106) est espacée de la première électrode externe (102) de sorte que la première tension à la première électrode externe (102) se décharge par l'intermédiaire de la seconde électrode externe (106) lorsque la première tension est appliquée.

2. Dispositif de traitement (2, 100) selon la revendication 1, dans lequel le dispositif de traitement (2, 100) comprend en outre un capteur (104).

3. Dispositif de traitement (2, 100) selon la revendication 2, dans lequel le capteur (104) est un capteur d'imagerie pour obtenir une ou plusieurs images en utilisant de la lumière passant dans le dispositif de traitement (2, 100).

4. Dispositif de traitement (2, 100) selon la revendication 2 ou 3, dans lequel le capteur (104) est destiné à détecter un ou plusieurs parmi : contact avec la peau, couleur de la peau, couleur des cheveux, grains de beauté, tatouages et affection de la peau.

5. Dispositif de traitement (2, 100) selon l'une quelconque des revendications 2-4, dans lequel la seconde électrode externe (106) est une boîte (110) dans laquelle le capteur (104) est disposé.

6. Dispositif de traitement (2, 100) selon la revendication 5, dans lequel la boîte (110) comprend un logement électriquement conducteur (112) pour le capteur (104) qui fournit un blindage contre les interférences électromagnétiques, EMI, dues à la première tension sur la première électrode externe (102).

7. Dispositif de traitement (2, 100) selon la revendication 6, dans lequel le capteur (104) est un capteur d'imagerie pour obtenir une ou plusieurs images en utilisant de la lumière entrant dans le dispositif de traitement (2, 100), dans lequel le logement électriquement conducteur (112) présente une extrémité ouverte à travers laquelle ladite lumière passe, et dans lequel la boîte (110) comprend en outre :
un prisme triangulaire (116) avec deux surfaces latérales triangulaires (120), une première surface, une seconde surface et une surface hypoténuse (118), dans lequel la première surface est agencée de manière adjacente à l'extrémité ouverte du logement électriquement conducteur (112) de sorte que de la lumière entrant dans le prisme triangulaire (116) au niveau de la seconde surface se réfléchit de la surface hypoténuse (118) vers la première surface, dans lequel la seconde surface est couverte d'une grille de nanofils conducteurs (122) qui agit comme un polariseur optique pour la lumière incidente, et dans lequel la surface hypoténuse (118) et les surfaces latérales triangulaires (120) sont revêtues de métal pour fournir un blindage du capteur d'imagerie contre les EMI dues à la première tension sur la première électrode externe (102).

8. Dispositif de traitement (2, 100) selon l'une quelconque des revendications 2-7, dans lequel au moins une partie de la seconde électrode externe (106) est plus proche de la première électrode externe (102) que toute partie du capteur.

9. Dispositif de traitement (2, 100) selon l'une quelconque des revendications 2-8, dans lequel le capteur est agencé au sein du dispositif de traitement (2, 100) pour détecter un ou plusieurs paramètres du sujet par l'intermédiaire d'une fenêtre de sortie de lumière (10) à travers laquelle l'impulsion de lumière est émise à partir du dispositif de traitement (2, 100).

10. Dispositif de traitement (2, 100) selon l'une quelconque des revendications 1-9, dans lequel la première électrode externe (102) est un composant métallique.

11. Dispositif de traitement (2, 100) selon l'une quelconque des revendications 1-10, dans lequel la première électrode externe (102) est un réflecteur pour guider l'impulsion de lumière vers la fenêtre de sortie de lumière (10).

12. Dispositif de traitement (2, 100) selon l'une quelconque des revendications 1-11, dans lequel la plus petite distance entre la seconde électrode externe (106) et la première électrode externe (102) est égale ou inférieure à une distance de rupture de l'air pour la première tension à pression atmosphérique.

13. Dispositif de traitement (2, 100) selon l'une quelconque des revendications 1-12, dans lequel la première tension est entre 12 et 17 kV.

14. Dispositif de traitement (2, 100) selon l'une quelconque des revendications 1-13, dans lequel la source de lumière (12, 101) est une lampe à décharge gazeuse.

15. Dispositif de traitement (2, 100) selon la revendication 14, dans lequel la lampe à décharge gazeuse est une lampe flash.

16. Dispositif de traitement (2, 100) selon l'une quelconque des revendications 1-15, dans lequel l'opération de traitement est de l'épilation par lumière pulsée intense, IPL.
